# EUROPÄISCHE PATENTANMELDUNG

(11) **EP 1 654 937 A1**
(43) Veröffentlichungstag der Anmeldung: **10.05.2006**
(21) Anmeldenummer: 05023960.7
(22) Anmeldetag: 03.11.2005
(51) Int. Cl.: A23L 1/223, A23L 1/30, A61K 36/54, A61K 36/42, A61P 3/10

(54) **Momordica charantia-Zimt-Zubereitung**

(30) Priorität: 05.11.2004 DE 202004017113 U
(71) Anmelder: Salus-Haus GmbH & Co. KG, 83052 Bruckmühl (DE)
(72) Erfinder: Greither, Otto, 83052 Bruckmühl (DE)
(74) Vertreter: Becker Kurig Straus

(57) **Zusammenfassung**

Die vorliegende Erfindung betrifft eine Kräuter-Zusammensetzung in Form einer Tablette oder eines Tees, die aus getrockneten und gegebenenfalls pulverisierten Kräutern, Samen, Rinden oder Wurzeln oder aus Extrakten aus Kräuter-Pflanzenteilen zusammengesetzt ist, wobei die Zusammensetzung eine Kräutermischung aus einer Kombination der Komponenten:
(a) Momordica charantia und
(c) Cinnamomum spec., insbesondere Cinnamomum ceylanicum sowie gegebenenfalls als Rest übliche pharmazeutischen Zusatz- und Hilfsstoffe
enthält und wobei sie zur besonderen Ernährung bei Diabetes mellitus im Rahmen eines Diätplanes einsetzbar ist.

## Beschreibung

Die vorliegende Erfindung betrifft eine Zusammensetzung aus pflanzlichen Bestandteilen, welche zur besonderen Ernährung bei Diabetes mellitus geeignet ist. Die vorliegende Erfindung betrifft daher eine Kräutermischung, die aus getrockneten und gegebenenfalls pulverisierten Kräutern, Pflanzenteilen, insbesondere Früchten oder Extrakten von Kräuter-Pflanzenteilen zusammengesetzt ist. Die erfindungsgemäße Mischung besteht aus getrockneten und gegebenenfalls pulverisierten Pflanzenteilen oder von Extrakten von Momordica charantia in Kombination mit Cinnamomum spec., insbesondere Cinnamomum ceylanicum oder Extrakten aus Zimtrinde und gegebenenfalls einer oder mehrerer Substanzen aus der Gruppe aus Lemongras, Pfefferminzblättern, Lindenblüten, Orangenschalen, Vanille oder Hagebuttenschalen oder Hibiscusfruchtkelchen, Ginkgoblättern, Kardamom, Süßholzwurzeln, Kakao sowie gegebenenfalls als Rest einer oder mehrerer Substanzen aus der Gruppe der Vitamine, Mineralstoffe, Spurenelemente etc.

In einer weiteren Ausführungsform der vorliegenden Erfindung liegt die Zusammensetzung in Form einer Tablette, insbesondere einer Film- oder Kautablette oder eines Tees vor. Falls die erfindungsgemäße Zubereitung in Form eines Tees vorliegt, so betrifft diese eine mit Wasser aufgieß- oder aufbrühbare Tee-Zubereitung, welche zur besonderen Ernährung bei Diabetes mellitus im Rahmen eines Diätplanes geeignet ist.

In allen Altersstufen ist Diabetes mellitus ein wachsendes gesundheitspolitisches Problem. In verschiedenen nationalen und übernationalen Programmen wird versucht, eine populationsbezogene Kontrolle und Prävention zu implementieren und die Versorgung chronisch Kranker zu verbessern. Die Programme gehen davon aus, Risikofaktoren zu eliminieren oder zu reduzieren. Die Risikofaktoren lassen sich in zwei Gruppen einteilen:
1. Übergewicht, Ernährung, eingeschränkte körperliche Aktivität, Alter, Schwangerschaft und genetische Disposition und
2. Hyperinsulinämie, Hyperglykämie, Dyslipidämie und Hypertonie.

In der multifaktoriellen Analyse sind Adipositas und Hyperinsulinämie die stärksten Prädiktoren für die Entwicklung des Diabetes mellitus Typ 2.

In letzter Zeit wird in der Literatur von einer Blutzucker senkenden Wirkung von Momordica charantia berichtet. Momordica charantia wird im deutschen Sprachraum Balsambirne, Bittergurke oder Bittermelone genannt. Diese Art gehört zur Familie der Kürbisgewächse und ist mit der Wassermelone verwandt. Momordica charantia kommt in Indien, China, den Philippinen, im tropischen Afrika, im mittleren Osten und in Amerika vor. Von der Pflanze werden die unreifen Früchte, die Samen, die Wurzeln und die Blätter verwendet.

Neben dem hohen Protein-, Mineralstoff- und Kohlenhydrat- sowie dem niedrigen Fettanteil werden Calcium, Karotin, Riboflavin, Vitamin A und C in Momordica charantia nachgewiesen. Aus Blättern, Samen und Frucht werden u. a. Triterpenglykoside (Momordicine und Momordicoside) isoliert. Von besonderem Interesse sind aber aus der Frucht und dem Samen gewonnene Inhaltsstoffe wie Charantin, einem Gemisch von β-Sitosterol-β-D-Glucosid und α-5,25-Stigmastadien-3-O-β-D-Glucosid im Verhältnis 1:1 mit vermuteter hypoglykämischer Wirkung. Weiterhin von besonderer Bedeutung ist p-Insulin mit hoher Homologie zu bovinem Insulin, aber immunologisch nicht kreuzreaktiv, welches bei Injektion ebenfalls Blutzucker senkende Wirkung zeigt. Weiterhin wurden in Momordica charantia die antiviralen Proteine MAP 30 und RIPs isoliert, wobei das Protein MAP30 in vitro die HIV-1 Infektion und die Virus-Replikation hemmt.

WO 98/43484 beschreibt im übrigen die Isolierung von aktiven Peptiden aus Momordica charantia, welche hypoglykämische Aktivität aufweisen sollen.

Aus Momordica charantia wurden beispielsweise auf viele verschiedene Arten Extrakte wie Heißwasserextrakte oder auch ethanolische Extrakte erzeugt. Weiterhin sind auch asiatische Kräutertees aus getrockneten Momordica charantia-Früchten im Handel erhältlich. Diese Gesundheitstees mit unterschiedlichsten Kräuterzusammensetzungen erfreuen sich großer Beliebtheit. Nachteilig bei diesen Tees ist jedoch die nicht überzeugende Wirkung bzw. der sehr bittere Geschmack.

Auch von Zimt ist eine Blutzucker senkende Wirkung bekannt. Ab der Mitte des 20. Jahrhunderts untersuchten Pharmakologen eingehend die Effekte des Zimts. Erst vor wenigen Monaten erschien das Ergebnis einer klinischen Doppelblindstudie, in deren Rahmen pulverisierte Zimtrinde an insgesamt 60 Patienten mit Typ-2-Diabetes getestet wurde. Die Patienten wurden dabei in sechs Gruppen eingeteilt, die 40 Tage lang täglich entweder 1, 3 oder 6 Gramm Zimtrinde oder Placebo erhielten. Nach den 40 Behandlungstagen waren die Blutzuckerspiegel in der Verumgruppe um 18 bis 29 Prozent gesenkt worden.

Weiterhin wurde der Einfluß von Zimtextrakt auf die Zuckerverwertung bei Ratten überprüft. Die von Wissenschaftlern der Nagoya Universität in Japan mit Zimtextrakt behandelten Tiere zeigten eine Normalisierung des Glucosestoffwechsels. Zimtextrakt verhindert die Entstehung einer Insulinresistenz, wie sie für Typ-2-Diabetes typisch ist.

Zimt ist ein Lorbeergewächs aus Südostasien. Die heute verwendeten Zimtarten werden in China, Sri Lanka, Indien und in Madagaskar angebaut.

Zimt hat auch eine entzündungshemmende und schmerzlindernde Wirkung. Wichtige Inhaltsstoffe von Zimt sind Cinnamaldehyde, Eugenol und Phellandren.

Der Erfindung liegt die Aufgabe zugrunde, eine Kräuter-Zusammensetzung mit weitgehend gesundheitlichen und physiologischen Wirkungen vorzuschlagen, die auch zur besonderen Ernährung bei Diabetes mellitus im Rahmen eines Diätplanes geeignet ist.

Diese Aufgabe wird durch die Kräuter-Zusammensetzung gemäß Anspruch 1 gelöst, die aus getrockneten und gegebenenfalls pulverisierten Kräutern, Samen, Rinden oder Wurzeln oder aus Extrakten von Pflanzenteilen zusammengesetzt ist und insbesondere zur Ernährung bei Diabetes mellitus im Rahmen eines Diätplanes geeignet ist. Die erfindungsgemäße Kräuter-Zusammensetzung besteht aus einer Mischung aus einer Kombination der Komponenten:
(a) Momordica charantia und
(b) Cinnamomum spec., insbesondere Cinnamomum ceylanicum sowie gegebenenfalls als Rest aus üblichen pharmazeutischen Zusatz- und Hilfsstoffen besteht, wobei die Zubereitung in Form einer Tablette oder eines Tees vorliegt.

In einer ganz besonderen Ausführungsform der Erfindung wird Zimtrindenpulver von Cinnamomum ceylanicum eingesetzt, da diese Spezies besonders cumarinarm ist.

In einer anderen Ausführungsform können auch Extrakte aus Momordica charantia-Früchten und auch gegebenenfalls von Extrakten aus Zimtrinde (Cinnamomum spec.) eingesetzt werden. Erfindungsgemäß bevorzugt sind hier wässerige oder wässerig alkoholische Extrakte aus den Pflanzenteilen, insbesondere von Momordica-Früchten oder von Zimtrinde. Die Momordica charantia-Früchte können frisch oder getrocknet vorliegen.

Die erfindungsgemäßen Extrakte können aus Momordica charantia-Früchten erhalten werden, umfassend die Schritte: Extraktion der ganzen oder zerkleinerten frischen oder getrockneten Momordica charantia-Früchte mit Wasser oder einem organischen Lösungsmittel aus der Reihe der aliphatischen C₁ bis C₄ Alkohole, Ketone, Ester, Ether, bevorzugt Ethanol, Methanol oder Gemische dieser Verbindungen mit Wasser, bei Raumtemperatur oder erhöhter Temperatur, bevorzugt bei 30 bis 60° C. Insbesondere wird erfindungsgemäß Wasser oder Gemische aus Ethanol und Wasser verwendet (0 bis 90 Vol. % Ethanol). Bei der Extraktion mit Wasser werden in der Regel für ein Teil Extrakt 2 bis 6 Teile getrocknete Pflanzenteile bzw. 20 bis 40 Teile frische Früchte benötigt. Die Extraktausbeute ist abhängig von der Qualität der Früchte, den Extraktionsbedingungen und dem verwendeten Auszugsmittel.

Weiterhin betrifft die vorliegende Erfindung auch die Verwendung von Extrakten aus Zimtrinde (Cinnamomum spec.).

Dieser erfindungsgemäße Extrakt kann durch ein Verfahren zur Herstellung obigen Extrakts aus Zimtrinde (Cinnamomum spec.) erhalten werden, umfassend die Schritte: Extraktion der ganzen oder zerkleinerten und getrockneten Zimtrinde (Cinnamomum spec.) mit Wasser oder einem organischen Lösungsmittel aus der Reihe der aliphatischen C₁ bis C₄ Alkohole, Ketone, Ester, Ether, bevorzugt Ethanol, Methanol oder Gemische dieser Verbindungen mit Wasser, bei Raumtemperatur oder erhöhter Temperatur, bevorzugt bei 60 bis 90° C. Insbesondere wird erfindungsgemäß Wasser oder ein Gemisch aus Ethanol und Wasser verwendet. Bei der Extraktion mit Wasser werden in der Regel für ein Teil Extrakt 4 bis 20 Teile Zimtrinde benötigt. Die Extraktausbeute ist abhängig von der Qualität der Zimtrinde, den Extraktionsbedingungen und dem verwendeten Auszugsmittel. Der Wassergehalt der resultierenden Trockenextrakte beträgt < 5,0 %. Der Polyphenolgehalt der Trockenextrakte beträgt 20 bis 30 % bei einem Gehalt an Cumarin von < 0,2 %.

Der erfindungsgemäßen Zubereitung können weiterhin eine oder mehrere Substanzen zugesetzt werden, ausgewählt aus der Gruppe, bestehend aus Nährstoffen, Antioxidantien, Geschmacksstoffen und Süßungsmitteln. Weiterhin können eine oder mehrere Substanzen zugesetzt sein aus der Gruppe aus Lutein, Lycopin, α- oder β- Karotin, Vitamin A, Vitamin B₁, Vitamin C, Vitamin E, Folsäure, Jod, Selen, Kupfer, Zink, Mangan, Chrom und weiteren Spurenelementen, Ubichinon (Coenzym Q). Sämtliche oben genannten Substanzen können auch als ihre Salze oder sonstigen Derivate eingesetzt werden.

In bevorzugten Ausführungsformen der Erfindung wird Vitamin E als R,R,R-α-Tocopherol und in Form von anderen aktiven Tocopherolen zugesetzt. Chrom kann z. B. als Chrom-III-chlorid und Jod als Kaliumjodid zugesetzt werden.

Die erfindungsgemäße Zubereitung liegt in Form einer Tablette oder eines Tees vor. Im Falle einer Tee-Zubereitung ist diese mit Wasser aufgieß- oder aufbrühbar. Die erfindungsgemäße Tee-Zubereitung besteht im wesentlichen aus einer Mischung aus 25 bis 80 Gew.-%, insbesondere aus 55 bis 65 Gew.-% Momordica charantia, insbesondere. aus getrockneten Momordica-Früchten und aus 20 bis 75 Gew.-%, insbesondere aus 20 bis 30 Gew.-% Zimtrindenpulver oder -extrakt sowie gegebenenfalls als Rest aus Lemongras und/oder Pfefferminzblättern.

Außerdem konnte bei der erfindungsgemäßen Teezubereitung z. B. mit Pflanzenteilen aus der Gruppe aus Hagebuttenschalen, Hibiscusfruchtkelchen, Pfefferminzblättern, Lindenblüten, Orangenschalen, Vanille, Ginkgoblättern, Lemongras, Kardamom, Süßholzwurzeln und Kakao eine besonders gute geschmackliche Abstimmung erzielt werden, die bei den Patienten hervorragend aufgenommen wurde.

In einer anderen Ausführungsform kann die Zubereitung in Form einer Tablette, d. h. einer Filmtablette oder einer Kautablette vorliegen. Bei einer Kautablette beträgt der Anteil an Momordica charantia z. B. bei 25 bis 32 Gew.-%, insbesondere bei 27 Gew.-%, 8 bis 12 Gew.-% Zimtrindenpulver oder -extrakt und als Rest finden sich gegebenenfalls weitere pharmazeutisch neutrale Zusatz- und Hilfsstoffe.

Falls die Zubereitung in Form einer Filmtablette vorliegt, beträgt der Gew.-%-Anteil an Bittermelonenfrucht 50 bis 60 Gew.-%, insbesondere 53,2 Gew.-% und 18 bis 22 Gew.-% Zimtrindenpulver.

Die Erfindung wird nun anhand der folgenden Beispiele näher erläutert.

### Beispiel 1: Herstellung von wässerigen Momordica charantia-Extrakten 2-stufig, Extraktionstemperatur 30° C

200 g getrocknete und zerkleinerte Momordica charantia-Früchte werden in einer 2-stufigen Bewegungsmazeration mit zusammen 2500 g Wasser bei 30° C (2 Std./2Std.) ausgezogen. Aus dem 1. Auszug (1500 g Wasser) resultieren 900 g Fluidextrakt mit einem Trockensubstanzgehalt von 4,5 % und einem Droge-Extrakt-Verhältnis von ca. 5:1. Der 2. Auszug (1000 g Wasser) ergibt weitere 900 g Fluidextrakt (Trockensubstanzgehalt 1,7 %, DEV 13,4:1). Beide Fluidextrakte werden zentrifugiert und vereinigt und ergeben einen Fluidextrakt mit einem DEV von 3,6:1. Nachfolgend wird der Fluidextrakt auf einen Trockensubstanzgehalt von ca. 30 bis 40 % schonend im Vakuum eingeengt und anschließend im Vakuum getrocknet.

### Beispiel 2: Herstellung von wässerigen Momordica charantia-Extrakten (2-stufig) 2-stufig, Extraktionstemperatur 60° C

100 g getrocknete und zerkleinerte Momordica charantia-Früchte werden in einer 2-stufigen Bewegungsmazeration mit zusammen 1300 g Wasser bei 60° C (1 Std./1Std.) ausgezogen. Aus dem 1. Auszug (800 g Wasser) resultieren 505 g Fluidextrakt mit einem Trockensubstanzgehalt von 4,2 % und einem Droge-Extrakt-Verhältnis von ca. 5:1. Der 2. Auszug (500 g Wasser) ergibt weitere 440 g Fluidextrakt (Trockensubstanzgehalt 1,8 %, DEV 13:1). Beide Fluidextrakte werden zentrifugiert und vereinigt und ergeben einen Fluidextrakt mit einem DEV von 3,6:1. Nachfolgend wird der Fluidextrakt auf einen Trockensubstanzgehalt von ca. 30 bis 40 % schonend im Vakuum eingeengt und anschließend im Vakuum getrocknet.

### Beispiel 3: Herstellung von wässerigen Zimt-Extrakten 2-stufig, Cinnamomum ceylanicum, Extraktionstemperatur 80° C

2000 g Zimtrinde (Cinnamomum ceylanicum) werden in einer 2-stufigen Bewegungsmazeration mit zusammen 24000 g Wasser bei 80° C (2 Std./2Std.) ausgezogen. Aus dem 1. Auszug (14000 g Wasser) resultieren 9028 g Fluidextrakt mit einem Trockensubstanzgehalt von 1,3 % und einem Droge-Extrakt-Verhältnis von ca. 17:1. Der 2. Auszug (10000 g Wasser) ergibt weitere 9296 g Fluidextrakt (Trockensubstanzgehalt 0,50 %, DEV 38:1). Beide Fluidextrakte werden zentrifugiert und vereinigt und ergeben einen Fluidextrakt mit einem DEV von 12:1. Nachfolgend wird der Fluidextrakt auf einen Trockensubstanzgehalt von ca. 30 bis 50 % schonend im Vakuum eingeengt und anschließend im Vakuum getrocknet.

### Beispiel 4: Herstellung von wässerigen Zimt-Extrakten 2-stufig, Cinnamomum ceylanicum, Extraktionstemperatur 60° C

100 g Zimtrinde (Cinnamomum ceylanicum) werden in einer 3-stufigen Bewegungsmazeration mit zusammen 1100 g Wasser bei 60° C (2 Std./2 Std.) ausgezogen. Aus dem 1. Auszug (500 g Wasser) resultieren 208 g Fluidextrakt mit einem Trockensubstanzgehalt von 1,58 % und einem Droge-Extrakt-Verhältnis von ca. 30:1. Der 2. Auszug (300 g Wasser) ergibt weitere 260 g Fluidextrakt (Trockensubstanzgehalt 0,87 %, DEV 44:1). Der 3. Auszug (300 g Wasser) ergibt weitere 278 g Fluidextrakt (Trockensubstanzgehalt 0,5 %, DEV 72:1). Alle Fluidextrakte werden zentrifugiert und vereinigt und ergeben einen Fluidextrakt mit einem DEV von 14:1. Nachfolgend wird der Fluidextrakt auf einen Trockensubstanzgehalt von ca. 30 bis 40 % schonend im Vakuum eingeengt und anschließend im Vakuum getrocknet.

### Beispiel 5: Herstellung von wässerigen Zimt-Extrakten 2-stufig, Cinnamomum cassia, Extraktionstemperatur 85° C

500 kg Zimtrinde (Cinnamomum cassia) werden in einer 2-stufigen Bewegungsmazeration mit zusammen 3500 kg Wasser bei 85° C (2 Std./2Std.) ausgezogen. Aus dem 1. Auszug resultieren 2772 kg Fluidextrakt mit einem Trockensubstanzgehalt von 2,54 %. Der 2. Auszug ergibt weitere 2588 kg Fluidextrakt (Trockensubstanzgehalt 1,09 %). Beide Fluidextrakte werden über einen Dekanter von Feststoffen befreit und vereinigt und ergeben einen Fluidextrakt mit einem DEV von 5:1. Nachfolgend wird der Fluidextrakt auf einen Trockensubstanzgehalt von ca. 30 bis 50 % schonend im Vakuum eingeengt und anschließend im Vakuum getrocknet.

### Beispiel 6: Filmtablette mit Momordica-charantia-Fruchtpulver und Zimtrindenpulver

Im folgenden soll die Zusammensetzung einer erfindungsgemäßen Filmtablette mit Momordica-charantia-Fruchtpulver und Zimtrindenpulver angegeben werden.

| **Inhaltsstoff** | **Masse eff.** |
|---|---|
| Momordica-charantia-Fruchtpulver | 625,000 mg |
| Zimtrindenpulver | 250,000 mg |
| Chrom | 0,010 mg |
| Zink | 2,500 mg |
| Selen | 0,025 mg |
| Jod | 0,025 mg |
| Vitamin B₁ | 1,250 mg |
| Siliciumdioxid | 20,000 mg |
| Reisstärke | 85,000 mg |
| Sorbit | 150,000 mg |
| Magnesiumstearat | 11,000 mg |
| Schellacklösung 28%* | 29,000 mg |
| Talkum* | 1,000 mg |

| | |
|---|---|
| *Aus der Schellacklösung plus Talkum resultiert eine Lackschicht von ca.10 mg. | |

Die Gesamtmasse ist auf eine tägliche Verzehrmenge von 2 x 2 Tabletten berechnet. Die Tablette kann Abmessungen von 8,5 mm x 20 mm mit oder ohne beidseitige Bruchkerbe aufweisen.

### Beispiel 7: Kautablette mit Momordica-charantia-Fruchtpulver und Zimtrindenpulver

Die Zusammensetzung einer erfindungsgemäßen Kautablette mit Momordica-charantia-Fruchtpulver und Zimtrindenpulver ist wie folgt:

| **Inhaltsstoff** | **Masse eff.** |
|---|---|
| Momordica-charantia-Fruchtpulver | 625,000 mg |
| Zimtrindenpulver | 250,000 mg |
| Chrom | 0,010 mg |
| Zink | 2,500 mg |
| Selen | 0,025 mg |
| Jod | 0,025 mg |
| Vitamin B₁ | 1,250 mg |
| Xylitol | 1100,000mg |
| Reisstärke vorverkleistert | 170,000 mg |
| Natürliches Aroma | q.s. |
| Siliciumdioxid gefällt | 35,000 mg |
| Langkettige Partialglyceride | 65,000 mg |
| Calciumstearat | 30,000 mg |

Die Gesamtmasse ist auf eine tägliche Verzehrmenge von 2 x 2 Kautabletten berechnet. Die Kautablette kann Abmessungen von 6,6 mm x 20 mm aufweisen.

### Beispiel 8: Filmtablette mit Momordica-charantia-Extrakt und Zimtrinden-Extrakt

Im folgenden soll die Zusammensetzung einer erfindungsgemäßen Filmtablette mit Momordica-charantia-Extrakt und Zimtrinden-Extrakt angegeben werden.

| **Inhaltsstoff** | **Masse eff.** |
|---|---|
| Momordica-charantia-Extrakt | 500,000 mg |
| Zimtrinden-Extrakt | 83,000 mg |
| Chrom | 0,010 mg |
| Zink | 2,500 mg |
| Selen | 0,025 mg |
| Jod | 0,025 mg |
| Vitamin B₁ | 1,250 mg |
| Siliciumdioxid | 20,000 mg |
| Reisstärke | 85,000 mg |
| Sorbit | 150,000 mg |
| Magnesiumstearat | 11,000 mg |
| Schellacklösung 28%* | 29,000 mg |
| Talkum* | 1,000 mg |

| | |
|---|---|
| *Aus der Schellacklösung plus Talkum resultiert eine Lackschicht von ca. 10 mg. | |

Die Gesamtmasse ist auf eine tägliche Verzehrmenge von 1 Tablette berechnet. Die Tablette kann Abmessungen von 8,5 mm x 20 mm mit oder ohne beidseitige Bruchkerbe aufweisen.

### Beispiel 9: Kautablette mit Momordica-charantia-Extrakt und Zimtrinden-Extrakt

Die Zusammensetzung einer erfindungsgemäßen Kautablette mit Momordica-charantia-Extrakt und Zimtrinden-Extrakt ist wie folgt:

| **Inhaltsstoff** | **Masse eff.** |
|---|---|
| Momordica-charantia-Extrakt | 500,000 mg |
| Zimtrinden-Extrakt | 83,000 mg |
| Chrom | 0,010 mg |
| Zink | 2,500 mg |
| Selen | 0,025 mg |
| Jod | 0,025 mg |
| Vitamin B₁ | 1,250 mg |
| Xylitol | 1100,000mg |
| Reisstärke vorverkleistert | 170,000 mg |
| Natürliches Aroma | q.s. |
| Siliciumdioxid gefällt | 35,000 mg |
| Langkettige Partialglyceride | 65,000 mg |
| Calciumstearat | 30,000 mg |

Die Gesamtmasse ist auf eine tägliche Verzehrmenge von 2 x 1 Kautabletten berechnet. Die Kautablette kann Abmessungen von 6,6 mm x 20 mm aufweisen.

### Beispiel 10: Teezubereitung

Die erfindungsgemäße Tee-Zubereitung besteht aus 55 bis 65 Gew.-% Momordica-Früchten und aus 20 bis 30 Gew.-% Zimtrindenpulver sowie als Rest aus Lemongras.

### Zubereitungsmethode der erfindungsgemäßen Tee-Zubereitung

Ein Eßlöffel voll Kräutertee mit einem Liter kochendem Wasser übergießen und sechs bis acht Minuten ziehen lassen.

Der Tee kann dann jeweils abgeseiht werden. Der zubereitete Tee wird gesüßt oder ungesüßt vorzugsweise jeweils nach den Mahlzeiten getrunken. Der erfindungsgemäße Kräutertee wird langfristig oder kurzmäßig zumindest über acht Wochen angewendet.

Die erfindungsgemäße Teezubereitung zeigte bei Einnahme von über acht Wochen bei Patienten, dass der Nüchternblutzuckerspiegel im Vergleich zum Ausgangswert beträchtlich zu senken war und dass sich auch das HbA_{1c} wesentlich erniedrigte.

## Patentansprüche

1. Kräuter-Zusammensetzung in Form einer Tablette oder eines Tees, die aus getrockneten und gegebenenfalls pulverisierten Kräutern, Samen, Rinden und/oder Wurzeln oder aus Extrakten aus Kräuter-Pflanzenteilen zusammengesetzt ist, **dadurch gekennzeichnet, dass** die Zusammensetzung eine Kräutermischung aus einer Kombination der Komponenten:
(a) Momordica charantia und
(b) Cinnamomum spec., insbesondere Cinnamomum ceylanicum sowie gegebenenfalls als Rest übliche pharmazeutischen Zusatz- und Hilfsstoffe enthält.

2. Kräuter-Zusammensetzung gemäß Anspruch 1, **dadurch gekennzeichnet, dass** sie zur besonderen Ernährung bei Diabetes mellitus im Rahmen eines Diätplanes einsetzbar ist.

3. Kräuter-Zusammensetzung gemäß Anspruch 1 oder 2, **dadurch gekennzeichnet, dass** als Extrakt Extrakte aus Momordica charantia-Früchten und Cinnamomum spec.-Pflanzenteilen oder Extrakte der ganzen oder zerkleinerten frischen oder getrockneten Pflanzenteile eingesetzt werden.

4. Kräuter-Zusammensetzung gemäß Anspruch 3, **dadurch gekennzeichnet, dass** als Extraktionsmittel zur Herstellung der Extrakte wenigstens ein Lösungsmittel aus der Gruppe, bestehend aus Wasser, aus der Gruppe der aliphatischen C₁ bis C₄ Alkohole, der Ketone, der Ester, der Ether, oder Gemische dieser Verbindungen, insbesondere Ethanol oder Methanol eingesetzt wird.

5. Kräuter-Zusammensetzung gemäß einem der Ansprüche 1 bis 3, **dadurch gekennzeichnet, dass** sie weiterhin eine oder mehrere Substanzen enthält, die ausgewählt sind aus der Gruppe bestehend aus Lutein, Lycopin, α- oder β-Karotin, Vitamin A, Vitamin B₁, Vitamin E, Folsäure, Selen, Kupfer, Zink, Mangan, Chrom, Jod, Ubichinon (Coenzym Q). Alle genannten Substanzen sind auch als ihre Salze oder sonstige Derivate einsetzbar.

6. Kräuter-Zusammensetzung gemäß Anspruch 1 bis 5, **dadurch gekennzeichnet, dass** sie weiterhin getrocknete und gegebenenfalls pulverisierte Pflanzenteile aus der Gruppe aus Hagebuttenschalen, Hibiscusfruchtkelchen, Pfefferminzblättern, Lindenblüten, Orangenschalen, Vanille, Ginkgoblättern, Lemongras, Kardamom, Süßholzwurzeln, Kakao enthält.

7. Kräuter-Zusammensetzung gemäß einem der vorhergehenden Ansprüche 1 bis 6, **dadurch gekennzeichnet, dass** die Mischung zusätzlich ätherische Öle und oder Aromastoffe enthält.

8. Kräuter-Zusammensetzung gemäß einem der vorhergehenden Ansprüche 1 bis 7, **dadurch gekennzeichnet, dass** sie in Form einer Tee-Zubereitung vorliegt und 25 bis 80 Gew.-% Momordica charantia, insbesondere 55 bis 75 Gew.-% Momordica charantia, 20 bis 75 Gew.-% Cinnamomum spec., insbesondere 20 bis 30 Gew.-% Cinnamomum spezies sowie gegebenenfalls als Rest pharmazeutisch neutrale Zusatz- und Hilfsstoffe enthält.

9. Kräuter-Zusammensetzung gemäß einem der vorhergehenden Ansprüche 1 bis 8, **dadurch gekennzeichnet, dass** sie zusätzlich 25 bis 32 Gew.-Teile, insbesondere 27,5 Gew.-Teile Geschmackskorrigentien enthält.

10. Kräuter-Zusammensetzung gemäß einem der vorhergehenden Ansprüche 1 bis 9, **dadurch gekennzeichnet, dass** sie in Form einer Kautablette vorliegt und 25 bis 32 Gew.-% Momordica charantia-Frucht, insbesondere 27 Gew.-% Momordica charantia-Frucht sowie 8 bis 12 Gew.-%, insbesondere 11 Gew.-% Cinnamomum spec., insbesondere Cinnamomum ceylanicum, enthält.

11. Kräuter-Zusammensetzung gemäß einem der vorhergehenden Ansprüche 1 bis 10, **dadurch gekennzeichnet, dass** sie in Form einer Tablette vorliegt und 50 bis 60 Gew.-% Momordica charantia-Frucht sowie 18 bis 22 Gew.-%, insbesondere 21 Gew.-% Cinnamomum spec., insbesondere Cinnamomum ceylanicum enthält.
